# EUROPEAN PATENT APPLICATION

(11) **EP 2 085 056 A2**
(43) Date of publication of application: **05.08.2009**
(21) Application number: 09005645.8
(22) Date of filing: 27.04.2007
(51) Int. Cl.: A61F 2/44

(54) **Dual composition vertebral fixation device**

(30) Priority: 28.04.2006 US 745895 P
(62) Divisional of application: 07008690.5
(71) Applicant: Concept Matrix, LLC, Orlando, FL 32801 (US)
(72) Inventor: Eckman, Walter, W., Tupelo MS 38801 (US)
(74) Representative: Bohnenberger, Johannes

(57) **Abstract**

A vertebral fixation device for insertion between a pair of adjacent cervical vertebrae includes a frame comprised of a first material. The frame has a generally rectangular proximal end and a generally rectangular distal end. The distal end is connected to the proximal end by at least one upper arch and at least one lower arch. The upper and lower arches are spaced apart from each other. The frame has a generally hollow interior, substantially open lateral sides and a substantially open proximal end. At least one of the upper and lower arches are spaced between the lateral sides of the frame.

## Description

### BACKGROUND OF THE INVENTION

The present invention relates generally to vertebral fixation or defect devices, and more particularly to a dual composition vertebral fixation device for insertion into an intervertebral space and combining the benefits of support strength and rapid fixation achieved with rigid material, such as titanium, with the advantages of polymeric materials that reduce imaging interference and artifacts.

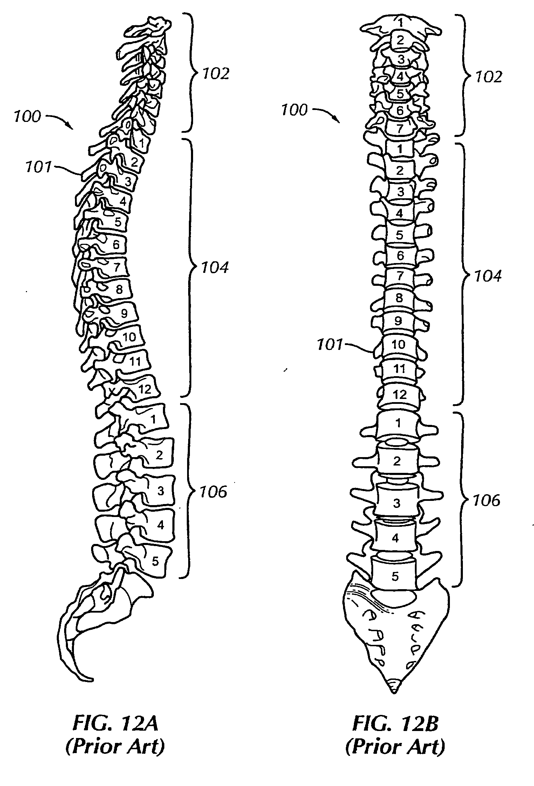

As shown in prior art Figs. 12A-17, it is known in the prior art that the spine 100, also known as the spinal column or vertebral column, supports the upper body, allows head, neck, and trunk motion, and includes twenty-four moveable vertebrae 101 including seven cervical vertebrae 102, twelve thoracic vertebrae 104, and five lumbar vertebrae 106, which extend from the skull to the sacrum.

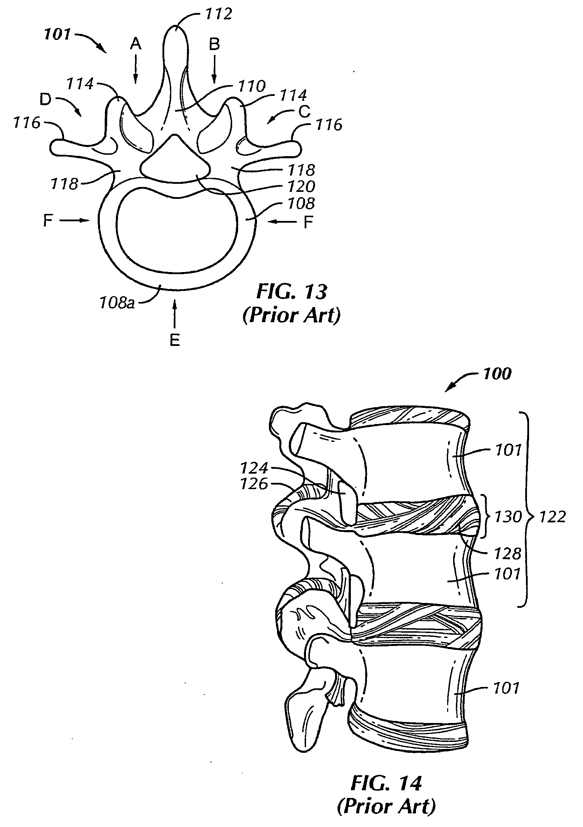

Referring to Fig. 13, with the exception of the first, uppermost cervical vertebra 102, each vertebra 101 has a vertebral body 108, a lamina 110, a spinous process 112, as well as facet structures 114 (which form facet joints), two transverse processes 116, and two pedicles 118, one on each side. Each individual vertebra 101 has a large foramen 120, which forms the spinal canal (not shown) when the vertebrae 101 are in their normal anatomical position forming the spine 100. The spinal cord and major nerve fiber groups pass through and are protected by the spinal canal. A strong fibrous membrane, the dura mater (not shown), also known as the dura, surrounds the spinal cord, nerve fibers, and fluid in the spinal canal.

Referring now to Figs. 13 and 14, each pair of adjacent vertebrae 101 along with interconnecting soft tissues and an intervetebral disk 128 constitutes a motion segment 122, also known as a functional spinal unit, and the combined motions of many such motion segments 122 constitute overall spinal motion at any one time. The joining of two vertebrae 101 also creates two neuroforaminae 124, also known as intervertebral foraminae, one on each side, each of which is bordered by a facet joint 126 dorsally, a pedicle 118 superiorly, a pedicle 118 inferiorly, and an intervertebral disk 128 ventrally. Each neuroforamina 124 allows passage of large nerve roots (not shown) and associated blood vessels (not shown). The intervertebral disk 128 resides in the space between adjacent vertebral bodies, the intervertebral space 130, also known as the interbody space or disk space. The level of each particular intervertebral space 130 and intervertebral disk 128 is identified by naming the vertebrae 101 superior and inferior to it, for example L 4-5 in the case of the intervertebral space 130 and intervertebral disk 128 between the fourth and fifth lumbar vertebrae 106.

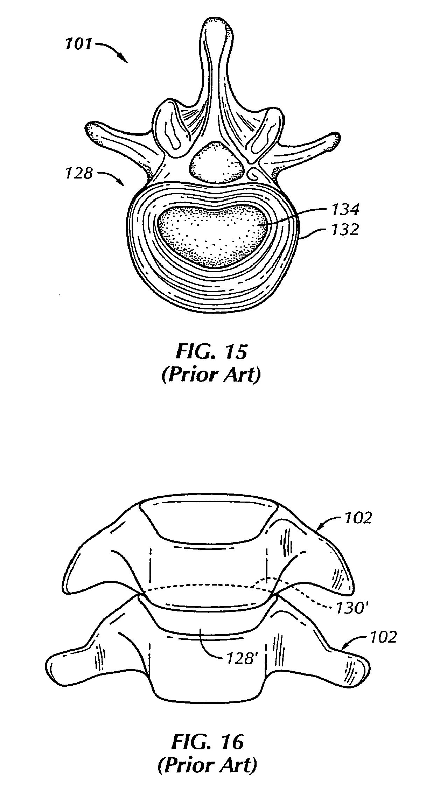

Referring to Fig. 15, each intervertebral disk 128 includes a collection of peripheral concentric rings of strong ligaments known as annular ligaments 132, also known as the annulus, and a softer central area of normally well hydrated material known as the nucleus 134. The annular ligaments 132 are arranged at different angles in alternate layers such that they provide support and stability, resisting excessive vertebral body rotation and axial motion when proper tension is maintained. Although described by some as a cushion, the nucleus 134 is relatively incompressible in a young healthy spine, and thus its major role is to provide support and tension of the annular ligaments 132 to maintain stability while allowing a limited range of motion.

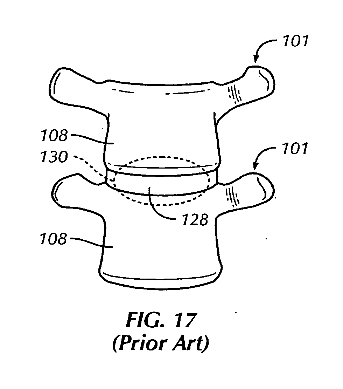

Referring now to Fig. 17, the superior surface and the inferior surface of the lumbar and thoracic vertebral bodies 108 are concave (the shape of the vertebral space 130 shown in phantom). Owing to the shapes of the inferior and superior surfaces of the vertebral bodies 108, the lumbar and thoracic intervertebral spaces 130 and intervertebral disks 128 are biconvex.

Referring now to Fig. 16, with the exception of the first cervical vertebra (not shown), in a cervical vertebra 102 the inferior surface of the vertebral body 108 is concave (as shown in phantom), while the superior surfaces are flatter centrally and curl laterally to form the uncinate processes, which partially articulate with the inferior surface of the vertebral body of the adjacent superior vertebra. Owing to the shapes of the inferior and superior surfaces of the vertebral bodies, the cervical intervertebral space 130' and intervertebral disk 128' are convex on the superior side but flatter on the inferior side. Cervical intervertebral disks 128' are generally flatter and thinner than lumbar and thoracic intervertebral disks 128.

Situations arise in which one or more motion segments 122 do not have adequate support or stability, which can lead to pain, deformity, stenosis of spinal canal or neuroforamina, and impairment or loss of nerve function. In some cases, surgical spine fusion is considered. Spine fusion is a process of growing bone between two or more adjacent vertebrae 101 such that the adjacent vertebrae 101 will move only in unison. This process involves placing bone, or material to guide or stimulate bone growth, in proximity to exposed bone of the vertebrae 101, and then allowing time for new bone to grow and form a structurally strong connection, or fusion, between the vertebrae 101. The earliest such procedures took place approximately a century ago, and the procedures have developed over many years, including various attempts to fuse posterior structures of the spine 100 such as the spinous process 112, lamina 110, facet joint 114, and transverse processes 116.

Recently, there has been more interest in fusion involving bone growth directly between adjacent vertebral bodies 101. Large amounts of well vascularized bone are in close proximity, there is a large surface area available, and the inherent compression force applied between vertebral bodies 101 by muscle tension and the upright position of the human body enhances bone formation and strength. The intervertebral disk space 130 has therefore become a major focus in interbody fusion surgery. The disk space 130 is cleared as much as possible, and cartilage and abnormal surface bone, also known as endplate bone, from adjacent vertebral bodies is removed, after which material is placed in the space to promote fusion. However, loose bone fragments do not provide structural support and therefore fusion is often unsuccessful. Structural bone grafts from the patient or donors have been successful, but may give rise to pain and complications if from the patient, and risk of disease transmission if from a donor.

Vertebral fixation devices are increasingly used to assist with fusion between vertebral bodies 108. Such devices are intended to provide support to prevent excessive collapse of space between vertebrae 101 which could result in stenosis of the spinal canal or neuroforamina, progressive deformity, impairment or loss of nerve function, or pain. Such devices also provide at least one compartment to fill with bone, or material which assists in bone growth, in order to maintain close contact with vertebral bone as new bone is encouraged to bridge across the space 130 involved.

Referring to Fig. 13, which shows a single plan view of a vertebra 101, it is known in the art that interbody devices can be inserted from several directions (indicated by arrowed lines) including posterior interlaminar approaches on both sides A, B, transforaminal or partially lateral approaches C, D, anterior approaches E, and straight lateral approaches F.

Efforts have been made to achieve stabilization with vertebral devices having ridges, threads, or grooves on cylinder shaped devices. Anterior approaches (E) allow more access to the disk space 130, but require more destruction of the annulus. In the lumbar spine 106, bilateral placement of multiple such devices has sometimes been necessary to achieve adequate stability. Cylindrically-shaped devices, inserted through posterior and transforaminal approaches (A, B, C, D) are associated with increased potential for nerve root or dural injury, particularly when drill tubes and reamers are used to prepare the disk space 130 for fusion.

Though vertebral fixation devices have proven useful in the lumbar or thoracic spine 104, 106, posterior and transforaminal placement (A, B, C, D) of any device is too dangerous in the cervical spine 102. Some cylindrical bone grafts and devices have also been associated with increased subsidence and kyphotic deformities, particularly in the cervical area. Subsidence is the sinking of devices or structural bone grafts into adjacent vertebral bodies.

Some cylindrical bone grafts and devices have also been associated with increased subsidence and kyphotic deformities. Subsidence is the sinking of devices or structural bone grafts into adjacent vertebral bodies. Lumbar fusion procedures involving posterior and transforaminal insertion have relied more on impacted devices, which do not provide immediate stabilization except by some degree of distraction, which is naturally lost by any subsidence, and they have been subject to excessive subsidence is some patients. It has been taught in the art that these devices should be inserted bilaterally to offer proper support.

Vertebral devices have been constructed with polymers such as PEEK and carbon fiber/PEEK combinations. Such devices have the advantage of minimal interference with future imaging studies whether by x-ray, CT scan, or MRI scan. Such devices usually have simple implanted metal markers in front and back to allow limited visualization of their position with x-rays or the like. They are made with thick, vertically straight walls to provide support strength, but once they subside a small amount the straight walls offer no effective resistance to excessive subsidence. The surface area provided for fusion is also limited by the thick walls. Polymer material in current use does not allow construction of sharp edges and fixation elements and does not allow for varied shapes which might solve many of the problems with subsidence.

In the cervical spine 102, it is known in the art that the addition of an anterior plate will limit subsidence and kyphotic deformity, but this adds cost and is not always successful. Complications such as backing out of fixation screws and screw and plate breakage have been significant problems. Anterior plates are difficult to install if more than two disk levels are fused, and may add to dysphagia. In addition, such a plate occupies much of the anterior surface of the superior and inferior vertebral bodies, and there is data to suggest that proximity of the plate to the adjacent disks promotes more rapid degenerative changes. If surgery is required at an adjacent level, it is almost always necessary to remove the plate to perform the surgery, which increases complexity and morbidity when further surgery is required.

Lumbar surgery is experiencing an evolution to minimally invasive surgery. This trend has led to the need for devices that can be inserted through small portals or working channels, usually through one small incision on one side of the patient's body. In lumbar fusion, vertebral devices should assist with rapid fixation to minimize the need for extensive additional fixation with bone screws, rods, and plates. Surgeons generally prefer posterior approaches for lumbar spine procedures due to the morbidity of anterior approaches, which also causes adhesion of major vessels and makes repeat anterior surgery very dangerous and even life threatening. When posterior lumbar fusion is performed, there is an opportunity to approach the spine 100 through the neuroforamina 124 and insert interbody devices through a small space free of vital structures which is located lateral to the dura in the canal, medial to the large nerve root passing through the neuroforamina 124, and bordered inferiorly or caudally by the pedicle 118 of the vertebra 101 below the involved disk space 130. The distance between peripheral edges of the vertebrae 101 at the disk space 130 is often small so that entry of devices has required considerable bone removal to safely impact devices into the disk space 130. If a device with a distal end of 3 mm or larger in height or transverse dimension is impacted into the disk space 130, it will frequently displace medially or laterally, and involve nerve structures. When working through a small portal or working channel, it is difficult to see this displacement, which makes some devices dangerous in this regard.

Conventional vertebral devices do not lend themselves to be used in minimally invasive surgery because open bilateral surgery is often necessary. When inserting a conventional vertebral device, a distraction tool is used to open the disk space on one side of the spine 100 to allow insertion of the vertebral device on the other side of the spine 100. Alternatively, pedicle screws on one or both sides of the spine 100 may be used with a distraction instrument to spread the disc space open for insertion of one or more vertebral devices.

It is therefore desirable to provide a vertebral fixation device designed to achieve rapid fixation while preventing excessive subsidence. In the cervical spine, the device should eliminate the need for ancillary stabilization devices such as anterior cervical plates and should be completely or nearly completely contained within the confines of the disk space. The device should have excellent support strength, but limit the amount of interference with future imaging studies. In the lumbar or thoracic spine, the device should reduce the potential for neural injury during insertion, and reduce or eliminate the need for bilateral lumbar pedicle screws. It is also desirable to provide a device that can be inserted during minimally invasive surgery and placed through an incision and directly into the disk space without manual or mechanical separation of the vertebrae and preferably asymmetrically obliquely across the disk space. It is further desirable for the device to have distinct elements that show its orientation and angulation in the disk space with minimal time and effort using imaging studies, such as with single plane lateral fluoroscopic x-ray.

### BRIEF SUMMARY OF THE INVENTION

Briefly stated, the present invention is directed to a dual composition vertebral fixation device for insertion between a pair of adjacent lumbar or thoracic vertebrae. The vertebral fixation device includes a frame comprised of a generally rigid first material for structural support strength and rapid fixation. The frame has an upper portion and a lower portion. The upper and lower portions are partially convex , spaced from each other and joined together by distal and proximal supports. A housing is comprised of a second material for minimizing interference with imaging. The housing has a convexly tapered distal end and two generally parallel side walls spaced from each other and extending from the tapered distal end. The housing is coupled to the frame by cooperatively surrounding at least a portion of the frame and oriented such that the side walls are generally perpendicular to the upper and lower portions of the frame when assembled.

In another aspect, the invention is directed to a vertebral fixation device for insertion between a pair of adjacent cervical vertebrae. The vertebral fixation device includes a frame comprised of a first material. The frame has a generally rectangular proximal end and a generally rectangular distal end. The distal end is connected to the proximal end by at least one upper arch and at least one lower arch. The upper and lower arches are spaced apart from each other. The frame has a generally hollow interior, substantially open lateral sides between the upper and lower arches and a substantially open proximal end.

In another aspect, the invention is directed to a method of installing a vertebral fixation device. The method includes the steps of: making an incision in a posterior region of a patient proximate a small gap between a first vertebra and a second vertebra of a spine of the patient, inserting a distal end of a surgical instrument through the small gap between the first and second vertebrae in order to access an intervertebral space between the first and second vertebrae, removing nuclear disk material from the intervertebral space; inserting the vertebral fixation device with protective covers, the vertebral fixation device having a frame comprised of a rigid first material for structural support strength and rapid fixation and a housing comprised of a second material for minimizing interference with imaging having a convexly tapered distal end, through the small gap, the tapered distal end separating the first and second vertebrae as the vertebral device is inserted into and within a nuclear region of the intervertebral space between the first and second vertebrae, and removing the protective covers to expose an interior of the vertebral fixation device and at least one projection and at least one sharpened edge extending from the vertebral fixation device.

In another aspect, the invention is directed to a method of installing a vertebral fixation device. The method includes the steps of: making an incision in an anterior region of a patient proximate a small gap between a first cervical vertebra and a second cervical vertebra of a spine of the patient, inserting a distal end of a surgical instrument and removing disk material from an intervertebral space between the first and second vertebrae, preparing the first and second vertebrae for fusion, inserting the vertebral fixation device with protective covers into the intervertebral disk space such that the proximal end of the vertebral fixation device is generally flush with an anterior edge of the first and second vertebrae, and removing the protective covers to expose an interior of the vertebral fixation device and the at least one projection extending from the vertebral fixation device.

### BRIEF DESCRIPTION OF THE SEVERAL VIEWS OF THE DRAWINGS

The foregoing summary, as well as the following detailed description of the preferred embodiments of the invention, will be better understood when read in conjunction with the appended drawings. For the purpose of illustrating the invention, there is shown in the drawings embodiments which are presently preferred. It should be understood, however, that the invention is not limited to the precise arrangements and instrumentalities shown.

In the drawings:

Fig. 1A is a front perspective view of a first preferred embodiment of a dual composition vertebral fixation device in accordance with the present invention;

Fig. 1B is a rear perspective view of the vertebral fixation device shown in Fig. 1A;

Fig. 1C is a right side elevation view of a frame of the vertebral fixation device shown in Fig. 1A;

Fig. 1D is a right side elevation view of a housing of the vertebral fixation device shown in Fig. 1A;

Fig. 2A is a top plan view of the vertebral fixation device shown in Fig. 1 A;

Fig. 2B is a top plan view of the frame shown in Fig. 2A;

Fig. 2C is a top plan view of the housing shown in Fig. 2A;

Fig. 3A is a cross-sectional view of the vertebral fixation device shown in Fig. 1A taken along line 3A-3A of Fig. 2A;

Fig. 3B is a cross-sectional view of the frame shown in Fig. 1C taken along line 3B-3B of Fig. 2B;

Fig. 3C is a cross-sectional view of the housing shown in Fig. 1D taken along line 3C-3C of Fig. 2C;

Fig. 4A is a top plan view of the vertebral fixation device shown in Fig. 1A with attached protective covers;

Fig. 4B is a right side elevation view of the vertebral fixation device and protective covers shown in Fig. 4A;

Fig. 5A is a front perspective view of a second preferred embodiment of a dual composition vertebral fixation device in accordance with the present invention;

Fig. 5B is a rear perspective view of the vertebral fixation device shown in Fig. 5A;

Fig. 6A is right side rear perspective view of a third preferred embodiment of a vertebral fixation device in accordance with the present invention;

Fig. 6B is a right side rear perspective view of the vertebral fixation device of Fig. 6A including an insert;

Fig. 7A is a right side elevation view of the vertebral fixation device shown in Fig. 6A;

Fig. 7B is a right side elevation view of the vertebral fixation device shown in Fig. 6B;

Fig. 8A is a top plan view of the vertebral fixation device shown in Fig. 6A;

Fig. 8B is a top plan view of the vertebral fixation device shown in Fig. 6B;

Fig. 9 is a right side rear perspective view of the vertebral fixation device shown in Fig. 6B with attached protective covers;

Fig. 10A is a right side elevation view of a fourth preferred embodiment of a vertebral fixation device in accordance with the present invention;

Fig. 10B is a right side elevation view of the vertebral fixation device of Fig. 10A including an insert;

Fig. 11 is a left side cross sectional view of the vertebral fixation device of Fig. 6A inserted into a vertebral disk space;

Fig. 12A is a right side elevation view of a human spinal column as is known in the art;

Fig. 12B is a front elevation view of a human spinal column as is known in the art;

Fig. 13 is a top plan view of a human vertebra as is known in the art;

Fig. 14 is a right side elevation view of a portion of the lumbar spine as is known in the art;

Fig. 15 is a top plan view of a lumbar vertebra as is known in the art;

Fig. 16 is a front view of a pair of cervical vertebrae as is known in the art; and

Fig. 17 is a front view of a pair of lumbar vertebrae as is known in the art.

### DETAILED DESCRIPTION OF THE INVENTION

Certain terminology is used in the following description for convenience only and is not limiting. The words "right," "left," "lower" and "upper" designate directions in the drawings to which reference is made. The words "inwardly" and "outwardly" refer to directions toward and away from, respectively, the geometric center of a dual composition vertebral fixation device in accordance with the present invention, and designated parts thereof. The words "convex" and "convexly shaped" refer to smooth or continuous surfaces as well as discontinuous surfaces or a plurality of flat planar surfaces which taken as a whole generally represent a convex shape. The phrase "partially convex" means that the feature taken as a whole has a generally convex shape and may include discontinuous or planar surfaces. The terminology includes the words noted above, derivatives thereof and words of similar import. Unless specifically set forth herein, the terms "a", "an" and "the" are not limited to one element but instead should be read as meaning "at least one".

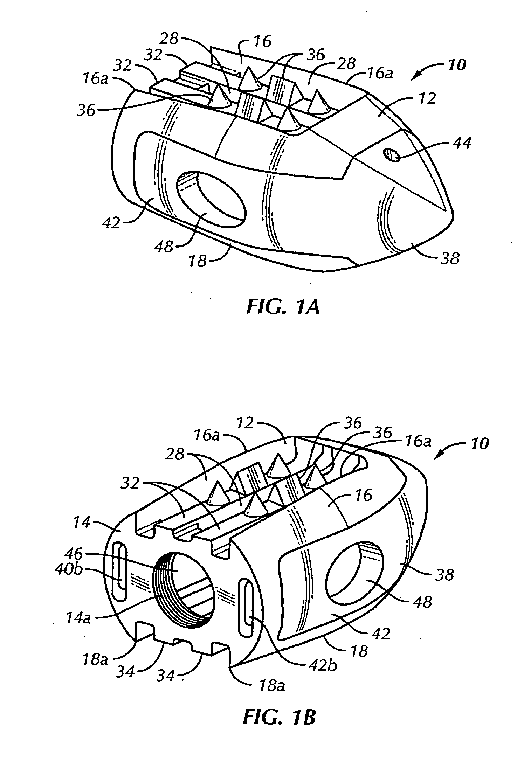

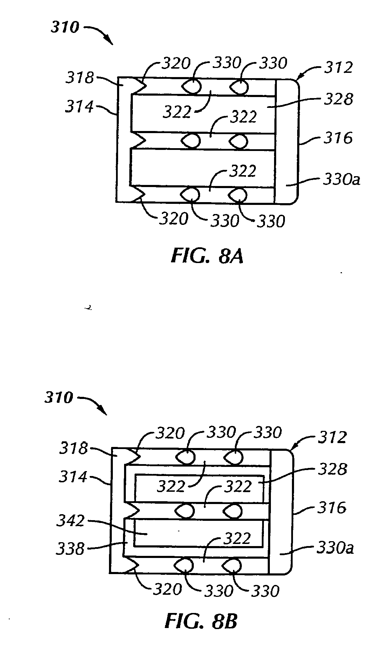

Referring to the drawings in detail, wherein like reference numerals indicate like elements throughout, there is shown in Figs. 1A-11 four dual composition vertebral fixation devices (vertebral fixation device or assembled vertebral fixation device), generally designated 10, 210, 310, 410 in accordance with first, second, third and four preferred embodiments respectively of the present invention for insertion between a pair of adjacent vertebrae 101. Unless specifically set forth herein, the description and method of each preferred embodiment also applies to the other preferred embodiments where applicable.

Referring to Figs. 1A-4B, the first embodiment of the vertebral fixation device 10 includes a frame 12 comprised of a first material. The frame 12 is preferably formed from a machining process, and the least amount of the first material to provide adequate compressive strength and mechanical stability is used. The first material is preferably constructed of surgical grade titanium and is discernable by electromagnetic imaging, such as x-rays and computed tomography (CT) scans, but has minimal interference with magnetic resonance imaging (MRI) scans. The first material may be selected from the group consisting of a biocompatible material such as machined bone, stainless steel, titanium, a cobalt-chrome alloy, a nickel plated metal, a biocompatible alloy, a biocompatible ceramic, a biocompatible polymeric material or a biologically absorbable material. Though the above materials are preferred, any material allowing adequate support strength that could be machined or milled into the shapes and features disclosed below could be used to form the frame 12 without departing from the spirit and scope of the invention.

The frame 12 includes a proximal support 14 located toward the proximal end of the vertebral fixation device 10. Upper and lower portions 16, 18 extend generally perpendicularly from the proximal support 14 and are spaced apart from and generally parallel to each other. The upper and lower portions 16, 18 are at least partially convexly shaped such that the height of the frame 12 is greatest at some location between the proximal and distal ends of the vertebral fixation device 10. Though an oval-shape as viewed from the proximal end is shown for the proximal support 14 and it is preferred that the upper and lower portions 16, 18 are convex, it is within the spirit and scope of the present invention that the proximal support 14 and upper and lower portions 16, 18 may have any other shape such as rectangular or diamond and be planar, concave or convex.

The proximal support 14 preferable includes a connector port 14a to temporarily and removably couple with an insertion tool (not shown) as described in further detail below. The connector port 14a is preferably a threaded hole but may also include any other structure for engaging with the insertion tool such as a socket, a detent, a hole a slot or the like.

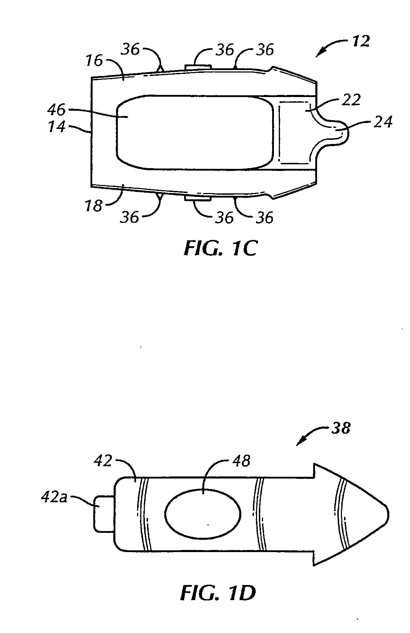

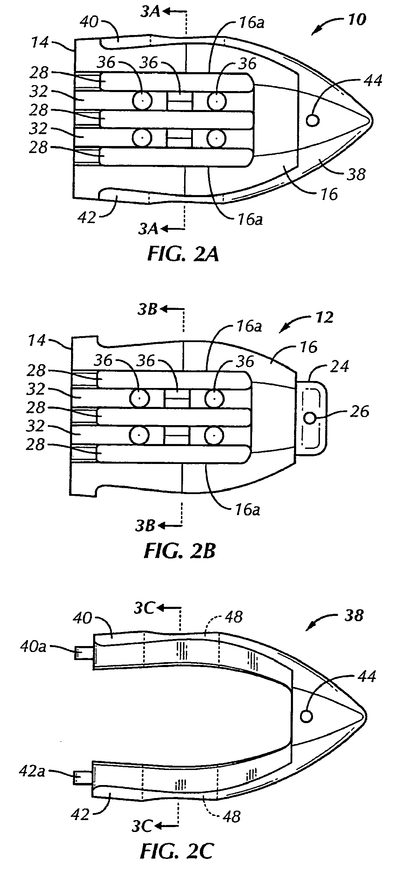

Referring to Figs. 1C and 2B, a distal support 22 extends between and supports the distal ends of the upper and lower portions 16, 18. The distal support 22 includes an extension 24. The extension 24 preferably projects distally from the rest of the frame 12. The extension 24 is preferably generally perpendicular to the distal support 22 but it is within the spirit and scope of the present invention that the extension 24 have a different orientation. The extension 24 has a first pin hole 26 extending vertically therethrough.

The upper portion 16 of the frame 12 includes at least one upper opening 28 and preferably three spaced and parallel upper openings 28. The lower portion 18 of the frame 12 has at least one lower opening 30 and preferably three spaced and parallel lower openings 30. The upper and lower openings 28, 30 are configured to allow bone growth into the intervertebral space 130 and are configured to align the vertebral fixation device 10 within the intervertebral space 130.

The frame 12 preferably includes at least one upper arch 32 and at least one lower arch 34 spanning the respective openings 28, 30. Preferably, the frame 12 includes two upper arches 32 and two lower arches 34. The upper and lower arches 32, 34 are generally parallel with respect to each other and are spaced apart. The upper and lower arches 32, 34 thicken in the vertical direction toward the proximal and distal ends of the frame 12. The proximal and distal supports 14, 22 help to add strength to the frame 12 and maintain a space between the upper and lower portions 16, 18.

Each of the upper and lower arches 32, 34 preferably includes at least one partially sharpened projection 36. There are preferably three projections 36 on each upper and lower arch 32, 34. The projections 36 are preferably conically or triangularly shaped and project outwardly from the arches 32, 34 by a predetermined distance. Alternatively, the projections 36 may slant toward the proximal end. The projections 36 are disposed at generally equally spaced intervals along each of the respective arches 32, 24. The projections 36 each act as a barb and assist with securely retaining the vertebral fixation device 10 in between a pair of vertebrae 101. Once the vertebral fixation device 10 is correctly in place and covers 50, discussed below, are removed, the projections 36 penetrate into the bone of the adjacent vertebrae 101 to resist motion of the vertebral fixation device 10 with respect to the adjacent vertebrae 101. The upper and lower portions 16, 18 preferably include at least one sharpened edge 16a, 18a respectively. Preferably, there are two sharpened edges 16a, 18a on the upper and lower portions 16, 18 which are parallel and project outwardly from the frame 12 and extend from the proximal end toward the distal end of the frame 12. The sharpened edges 16a, 18a of the upper and lower portions 16 and 18 and the projections 36 provide for rapid fixation of adjacent vertebral bodies thus achieving instant stabilization. Though a combination of conical and triangular projections 36 as shown in Figs. 1A-4B are preferred, the projections 36 may have any shape and may or may not be included on each arch 32, 34. Any number of projections 36 or sharpened edges 16a, 18a may be made in any number of sizes, shapes, and may be placed in any number of arrangements, so long as the requisite retaining and fixation function is achieved, without departing from the spirit and scope of the invention. In particular, such projections 36 as well as the shape and orientation of the remaining features of the frame 12 are shaped and arranged in ways that impart to the device a readily identifiable unique pattern when imaged after placement in the human body.

A housing 38 comprised of a second material has a convexly tapered distal end. The second material is preferably a polymeric material such as PEEK. However, any biologically compatible polymer or other material which does not interfere with imaging could be used. The housing 38 includes two generally parallel sides wall 40, 42 which are spaced from each other and extend from the tapered distal end of the housing 38. The housing 38 is coupled to the frame 12 by cooperatively surrounding at least a portion of the frame 12 and may be further secured by use of a pin, screws or any other known fastener. The housing 38 is oriented such that the side walls 40, 42 are generally perpendicular to the upper and lower portions 16, 18 of the frame 12 when the housing 38 and the frame 12 are assembled together.

Referring to Figs. 1B, 1D and 2C, the housing 38 is preferably separately constructed from the frame 12 and then assembled by snap-fitting or press-fitting with tabs 40a, 42a extending from the respective side walls 40, 42 and mating with corresponding detents 40b, 42b within the proximal support 14. The tabs 40a, 42a help to keep the side walls 40, 42 from extending laterally or otherwise out of contact with the frame 12. Fasteners (not shown) may be used to further secure the side walls 40, 42 to the frame 12.

Referring to Figs. 1A- 4B, the distal end of the housing 38 includes a second pin hole 44 extending vertically through the distal end. When the vertebral fixation device 10 is assembled, the second pin hole 44 is aligned with the first pin hole 26. A pin (not visible) with a length greater than the thickness of the extension 24 and less than the length of the second pin hole 44, is inserted within the first and second pin holes 26, 44 such that the pin retains the housing 38 to the frame 12. Along with the tabs 40a, 42a and or additional fasteners, the pin prevents the housing 38 from inadvertently detaching from the frame 12.

The side walls 40, 42 of the housing 38 may each include a generally circular side opening 48 which extends from the interior space 46 through the lateral sides walls 40, 42 to allow for bone growth between the interior space 46 and the intervertebral space 130. Although two generally circular openings 48 are shown, it is understood that more or less than two openings and/or openings of a different shape could be used without departing from the spirit and scope of the invention.

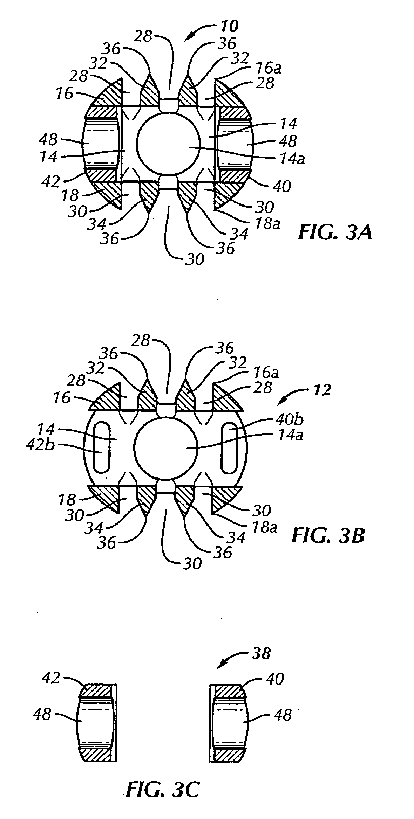

Referring to Figs. 1A and 1B, the assembled vertebral fixation device 10 has generally convex features such that the side walls 40, 42 toward the center of the vertebral fixation device 10 are the widest part of the vertebral fixation device 10 which then taper toward and are flush with the upper and lower portions 16, 18 and also taper toward the proximal and distal ends. A transverse cross sectional view of the assembled vertebral fixation device 10, as shown in Fig. 3A, is noncircular even when the covers 46 are inserted. The height of the assembled vertebral fixation device 10 is different from the width of the assembled vertebral fixation device 10. The vertebral fixation device 10 is therefore generally egg-shaped having a generally blunt proximal support 14 and a more pronounced pointed or tapered distal end. For insertion purposes, the proximal end of the vertebral fixation device 10 has a lesser average radius of curvature than the distal end. It is preferred that vertebral fixation device 10 have a generally egg-shaped or partially convex configuration but it is within the spirit and scope of the present invention that the vertebral fixation device 10 have a different shape such as rectangular or having both the proximal and distal end being tapered and having generally flat planar side walls 40, 42.

An outer profile of the vertebral fixation device 10 is characterized by a relatively gradual slope, such that the largest transverse dimension (height or width) of the vertebral fixation device 10 preferably changes no more than about 2 mm for every 1 mm change in length.
Preferably the distal end, in particular, has a slope that changes by no more than 2 mm for every 1 mm change in length. The distal end is relatively small, for example, the height or width preferably less than 2.5 mm in transverse dimension over the terminal 1mm of the distal end along the longitudinal axis or approximately 15-20% of the maximum height or maximum width of the vertebral fixation device 10. However, the distal end should not be so pointed such that it would easily drive through or penetrate the annulus on the opposite side of the intervertebral disk space 130. The size and taper or slope of the distal end of the vertebral fixation device 10 is intended to allow it to be impacted into the disk space while providing distraction of the periphery of the vertebral bodies to permit entry into the nuclear center of the disk. This minimizes a need to remove peripheral vertebral bone thus assisting with device retention and helps prevent potential extrusion of the vertebral fixation device 10. The vertebral fixation device 10 may be dimensioned in accordance with the requirements of specific applications, and other dimensional characteristics of the vertebral fixation device 10 are included within the scope of the present invention.

The assembled vertebral fixation device 10 preferably includes an interior space 46 generally defined by the shape of the assembled frame 12 and housing 38. The interior space 46 is preferably in communication with the upper and lower openings 28, 30. The interior space 46 may house bone grafts or non-bone matter to promote fusion.

The length of the assembled vertebral fixation device 10 as measured between the proximal and distal ends is preferably greater than the width of the vertebral fixation device 10 as measured between the lateral sides walls 40, 42 of the housing 38. The length of the vertebral fixation device 10 is also preferably greater than the height of the vertebral fixation device 10 as measured between the upper and lower portions 16, 18. The length of the assembled vertebral fixation device 10 as measured between the distal end of the housing 38 and the proximal support 14 of the frame 12 is preferably approximately 10 to 30mm. The width of the vertebral fixation device 10 as measured between the lateral side walls 40, 42 is preferably approximately 10mm to 25mm. The height of the vertebral fixation device 10 as measured between the upper and lower portions 16, 18 is preferably approximately 5 to 25mm.

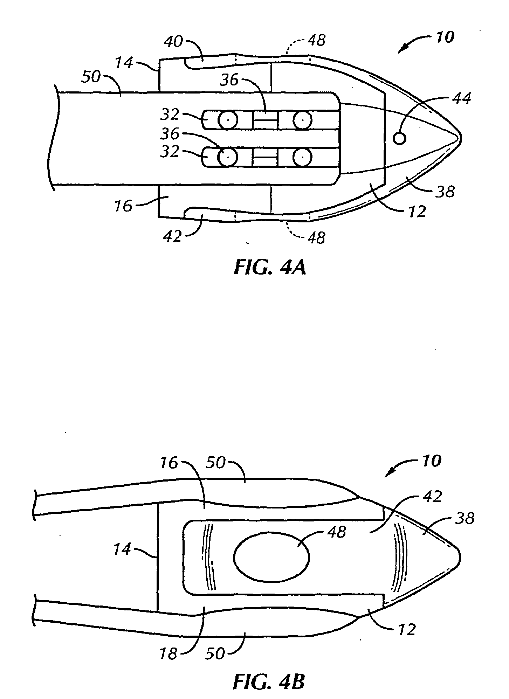

Referring to Figs. 4A and 4B, the vertebral fixation device 10 may include one or more protective covers 50. The covers 50 are inserted over the upper and lower openings 28, 30 in the upper and lower portions 16, 18 and cover the upper and lower openings 28, 30 during insertion of the vertebral fixation device 10 and limit the exposure of the projections 36. The covers 50 prevent debris from entering the upper and lower openings 28, 30 and the interior space 46 during insertion of the vertebral fixation device 10 and protect nearby anatomic structures, especially the nerve roots, dura, and vessels from injury by the projections 36. During insertion of the vertebral fixation device 10 into the disk space using an insertion tool (not shown), two pairs of protective covers 50 are preferably used to cover the upper and lower openings 28, 30. Each of the protective covers 50 is identical and is generally in the form of a smooth surfaced fork. The protective covers 50 are of a thickness that matches the height of the projections 36 above the arches 32, 34 and the sharpened edges 16a, 18a of the upper and lower portions 16, 18 so that preferably no sharp point or surface of the projections 36 or sharpened edges 16a, 18a protrudes beyond the smooth outer surface of the protective covers 50. The vertebral fixation device 10 with covers 46 inserted, forms a generally smooth or continuous convex top and bottom.

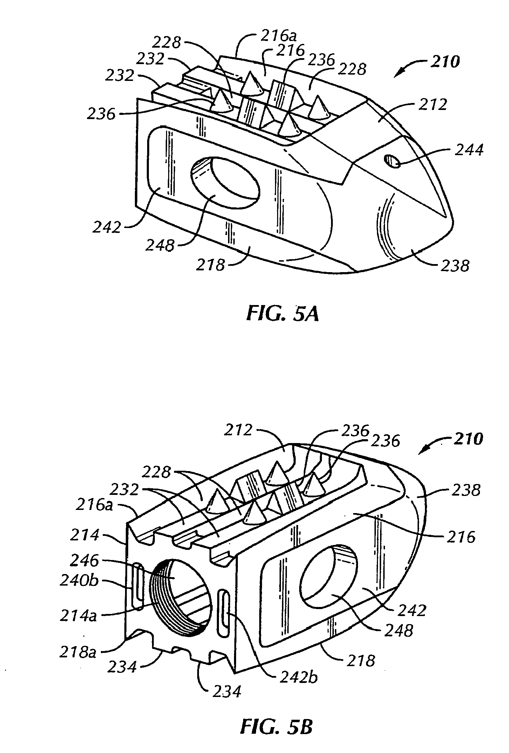

Referring to Figs. 5A-5B, the second preferred embodiment of the vertebral fixation device 210 includes generally planar side walls 240, 242 and has a tapered and convexly shaped distal end. The vertebral fixation device 210 of the second preferred embodiment is nearly identical to the first embodiment of the vertebral fixation device 10 except that the side walls 240, 242, and lateral sides of the upper and lower portions 216, 218 and proximal support 214 are generally flat or planar and rectangular in shape. Similar numbers indicate similar elements as discussed above for the first embodiment. A discussion of the similar features has been eliminated for convenience only and is not limiting. Though entirely planar side walls 240, 242 are shown, the side walls 240, 242 may be only partially planar such that a portion of the side walls 240, 242 and the lateral sides of the upper and lower portions 216, 218 retain a convex shape. The partially planar sides walls 240, 242 may allow for a more central insertion of the vertebral fixation device 210. The upper and lower portions 216, 218 preferably remain partially convex as viewed from the lateral sides similar to the first embodiment. Though a vertebral fixation device 210 having at least partially planar side walls 240, 242 is preferred, it is within the spirit and scope of the present invention that the side walls 240, 242 and lateral sides of the upper and lower portions 216, 218 form any shape.

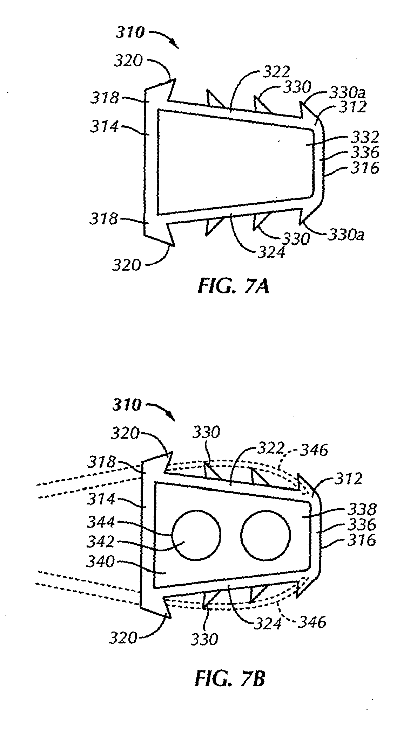

During the insertion procedure an incision is made in a posterior region of a patient proximate a small gap between a first vertebra 101 and a second vertebra 101 of a spine 100 of the patient. The incision is preferably between approximately 10 mm to 100 mm in span. The small gap is preferably off-center with respect to the posterior-side of the spine of the patient and proximate to the foraminae 120 of the first and second vertebrae (see directions C and D in Fig. 7) for the first embodiment. Alternatively, the incision may also be generally proximate the midline of the posterior-side of the spine for insertion of the vertebral fixation device 210 in a direction A or B. A working channel is then inserted through the incision. A distal end of a surgical instrument is inserted through the working channel between the first and second vertebrae 101 in order to access an intervertebral space 130 between the first and second vertebrae 101. Nuclear disk material 134 is removed from the intervertebral space 130. The vertebral fixation device 10, 210 with protective covers 50 is inserted through the small gap. The tapered distal end separates the first and second vertebrae 101 as the vertebral fixation device is inserted into and within a nuclear region 134 of the intervertebral space 130 between the first and second vertebrae 101. The positioning of the vertebral fixation device 10 is directed by the tilt and angle of the frame 12. The upper and lower portions 16, 18, proximal and distal supports 14, 22 and the various projections 36 help display how the vertebral fixation device 10 is positioned within the intervertebral space 130.

Once the vertebral fixation device 10 is inserted and properly positioned, the protective covers 50 are withdrawn, allowing penetration of the projections 36 and sharpened edged 16a, 18a into the vertebral bodies 108, and bringing vertebral bone into proximity with bone growth material in the interior space 46. The insertion tool (not shown) remains attached to the vertebral fixation device 10 during removal of the covers to maintain position, and is then removed when fixation is achieved.

Though the above method for installing the vertebral fixation device 10 is preferred, it is within the spirit and scope of the present invention that additional steps or a different order of the steps presented above be practiced.

The insertion tool (not shown) may be formed of any substantially rigid material, but preferably is formed of titanium, hardened stainless steel, or a biocompatible alloy, composite, polymeric material or the like of sufficient strength. It should be noted that the material of construction of the insertion tool (not shown) could be any material without diverging from the broad scope of the present invention. The protective covers 50 are preferably made of a biocompatible polymer that is strong and somewhat flexible. However, other materials could be used, such as low density metal alloys, without departing from the spirit or scope of the invention.

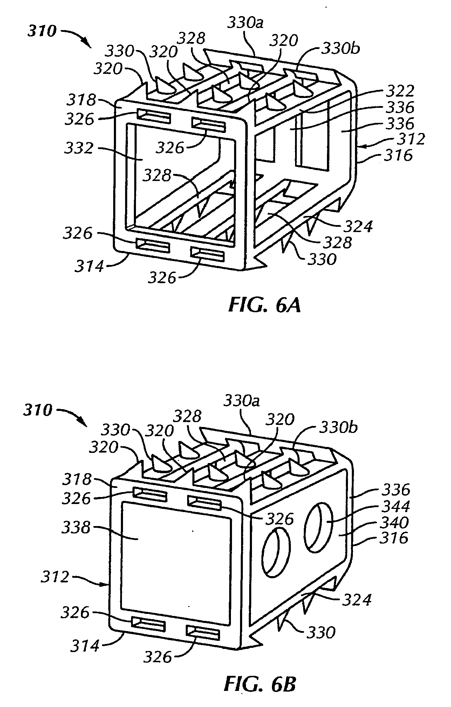

Referring now to Figs. 6A-9 and 11 the third preferred embodiment of the vertebral fixation device 310, for insertion between a pair of adjacent cervical vertebrae 102 includes a frame 312 comprised of a first material. The frame 312 is preferably formed from a machining process, and the least amount of the first material to provide adequate compressive strength and mechanical stability is used. The first material is preferably a surgical grade titanium and is discernable by electromagnetic imaging, such as x-rays and computed tomography (CT) scans, but has minimal interference with magnetic resonance imaging (MRI) scans. The first material may be selected from the group consisting of a generally rigid biocompatible material such as machined bone graft, titanium, a nickel plated metal, a biocompatible alloy, a biocompatible ceramic, a biocompatible polymeric material or a biologically absorbable material. Though the above materials are preferred, any material allowing adequate support strength that could be machined, milled or otherwise formed into the shapes and features disclosed below and that would have minimal interference with imaging studies could be used without departing from the spirit and scope of the invention.

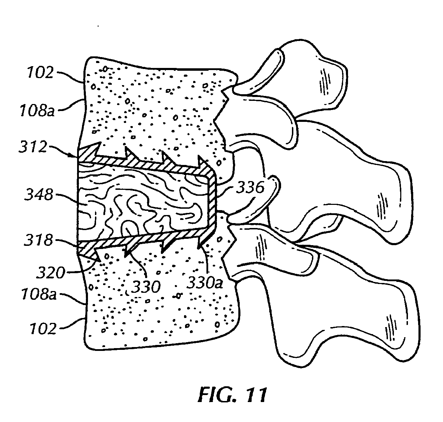

The frame 312 includes a generally rectangular proximal end 314 and a generally rectangular distal end 316. The proximal end 314 and distal end 316 may be rounded to form more of an oval or circular shape. The proximal end 314 preferably includes flanges 318 vertically extending above and below the remainder of the frame 312. The flanges 318 preferably extend a minimal amount past the proximal end 314 in the vertical direction and preferably not in the lateral direction. Because the flanges 318 do not extend laterally outwardly more than a maximum width of the vertebral fixation device 310, the lateral field of view is not obstructed during insertion of the vertebral fixation device 310. The flanges 318 act as a stopper to prevent excessive distal placement or migration of the vertebral fixation device 310, to help eliminate the risk of spinal canal encroachment and to aid in fixation. The flanges 318 abut the anterior edge of the vertebrae 102 being fused together when the vertebral fixation device 310 is installed as shown in Fig. 11. The flanges 318 may each include at least one projection 320. The projections 320 preferably point toward the distal end 316 of the vertebral fixation device 310 in order to engage with the vertebrae 102 above and below the vertebral fixation device 310. The projections 320 may be comprised of a plurality of distally pointing conical spikes as shown or may be an elongated edge extending at least partially along the flange 318. For example, the outer periphery of the flange 318 may be sharpened, curved or pointed to form the projections 320.

The flanges 318 and the proximal end 314 may be partially curved laterally with the convexity toward the proximal end 314 to match or better fit the contour or curve of the anterior leading edges of the adjacent vertebrae 102 once the vertebrae 102 are prepared for fixation. When the vertebral fixation device 310 is fully inserted (see Fig. 11), the flanges 318 are preferably flush, or nearly flush, with the anterior edges of the vertebrae 102 above and below the vertebral fixation device 310 so that there is minimal, if any, of the vertebral fixation device 310 protruding anterior to the adjacent vertebrae 102.

The distal end 316 of the vertebral fixation device 310 is connected to the proximal end 314 by at least one upper arch 322 and at least one lower arch 324. Preferably, the vertebral fixation device 310 includes three upper arches 322 and three lower arches 324 that are generally parallel and spaced apart. Between or proximate each arch 322, 324 is a gap 328 configured to allow bone growth into the intervertebral space 130. Four slots 326 preferably extend through the flanges 318, two spaced apart slots 326 for each flange 318. The slots 326 are generally aligned with the gaps 328. Though three arches 322, 324 and two gaps 328 are shown and described, it is within the spirit and scope of the invention that a single arch or planar segment with apertures of any geometry, or additional arches and gaps be used to connect the proximal and distal ends 314, 316.

Each of the arches 322, 324 preferably includes at least one and preferably two partially sharpened projections 330. The projections 330 are preferably conically shaped and extend outwardly from the arches 322, 324 and slant toward the proximal end 314. The projections 330 are preferably disposed at equally spaced intervals along each of the respective arches 322, 324. The top and bottom of the distal end of the vertebral fixation device 310 also preferably each include a sharpened edge 330a. The sharpened edge 330a is preferably a laterally extending edge that also slants toward the proximal end 314. The sharpened edges 330a extend both from the top and bottom of the distal end 316. The sharpened edges 330a may include receiving slots 330b which are aligned with the gaps 328. The sharpened edges 330a, with or without receiving slots 330b, provide a smooth fit and transition to covers 346 (described below). The sharpened edge 330a along with inserted covers 346, allow for smooth insertion of the vertebral fixation device 310.

The projections 330 and sharpened edge 330a each act as a barb and assist with securely retaining the vertebral fixation device 310 in place between a pair of vertebrae 102. Once the ) vertebral fixation device 310 is inserted in place and the covers 346 are removed, the proximal slant of the projections 330 and sharpened edges 330a penetrate into the bone of the adjacent vertebrae 102 to resist extrusion of the vertebral fixation device 310 and provide rapid fixation and stabilization of the adjacent vertebrae 102 to promote fusion. Though conical projections 330 are preferred, the projections 330 may have any shape such as an elongated triangle or edges and may or may not be included on each upper and lower arch 322, 324. Any number of projections 330 may be made in any number of shapes, and in any number of arrangements, so long as the requisite retaining and fixation function is achieved, without departing from the spirit and scope of the invention.

The frame 312 has a generally hollow interior 332 and substantially open lateral sides between the upper and lower arches 322, 324. The proximal end 314 is also substantially open. The generally hollow interior 332 forms an open interior chamber that is generally defined by the shape of the frame 312. The interior 332 may house bone grafts or non-bone matter to aid in fusion of the adjacent vertebrae 102. Fig 11 shows the inserted cervical fixation device 310 with bone 348 after healing.

The frame 312 preferably tapers from the proximal end 314 toward the distal end 316 between the upper and lower arches 322, 324 such that the distance between the upper and lower arches 322, 324 decreases from the proximal end 314 toward the distal end 316. The height of the proximal end 314 as measured between the top and the bottom is greater than the height of the distal end 16 as measured between the top and the bottom to maintain lordotic angulation of the vertebral bodies above and below the vertebral fixation device 310. This results in a generally trapezoidal shape when viewed from the side, as shown in Figs. 7A and 7B.

The length of the frame 312 as measured between the proximal and distal ends 314, 316 is preferably between approximately 10 mm and 14 mm. The width of the frame 312 as measured between the lateral sides of the frame 312 is preferably between approximately 10 mm to 14 mm. The height of the vertebral fixation device as measured between the upper and lower arches 322, 324 is the height necessary to fit between vertebral bodies 108 which is approximately 5 mm to 12 mm for a single level. The height could be increased if more than one level is needed to partially replace a damaged vertebral body 108.

The distal end 316 may include two or more generally spaced apart vertical members 336, identical and generally parallel to each other for supporting the distal ends of the upper and lower arches 322, 324. Preferably, each vertical member 336 connects an upper arch 322 to a corresponding lower arch 324. Though the use of a vertical member 336 is preferred, it is within the spirit and scope of the present invention that the distal end 316 have a different configuration such as a solid wall, a plurality of apertures of any geometric shape or be entirely open.

Referring to Figs. 6B, 7B, 8B and 9, the vertebral fixation device 310 may also include an insert 338. The insert 338 is comprised of a second material. The second material is preferably a radiotranslucent polymeric material such as PEEK. However, any biologically compatible polymer or other material which minimally interferes with imaging could be used. The insert 338 is positioned in the interior 332 of the frame 312 between the upper and lower arches 322, 324. The insert 338 forms a trapezoid or box-shaped structure corresponding to the shape of the interior 332. The insert 338 is generally in the form of a square in top plan view and trapezoidal or rectangular in side view. The insert 338 has an open central cavity 342, a generally open top and bottom and a generally closed proximal and distal ends. The insert 338 is disposed within at least a portion of the frame 312 and has lateral sides 340 exposed at least partially through the open lateral sides of the frame 312. The lateral sides 340 are generally flat with rounded or contoured edges on their surfaces. The lateral sides 340 extend generally parallel with respect to each other, surround the central cavity 342, and are preferably spaced apart along the outline of the shape which precisely complements the upper and lower arches 322, 324. Each lateral side 340 contains at least one but preferably two generally circular side openings 344 which extend from the central cavity 342 through the lateral sides 340 to allow for bone growth between the central cavity 342 and the intervertebral space 130'. Although two generally circular openings 344 are shown, it is understood that more or less than two openings of different shapes could be used without departing from the spirit and scope of the invention.

The insert 338 is preferably separately constructed from the frame 312 and then assembled by snap-fitting or press-fitting with suitable mating detents, grooves, edges or the like. The vertebral fixation device 310 could alternatively not include the insert 338 (Fig. 11). Bone could alternatively be used in place of the insert 338.

Surgical bone screws (not shown) could be inserted through additional holes (not shown) and/or the slots 326 in the flange 318 to further secure the vertebral fixation device 310 between the vertebral bodies in lieu of or in combination with the sharpened edges 320. Other fastening devices or the like could also be utilized.

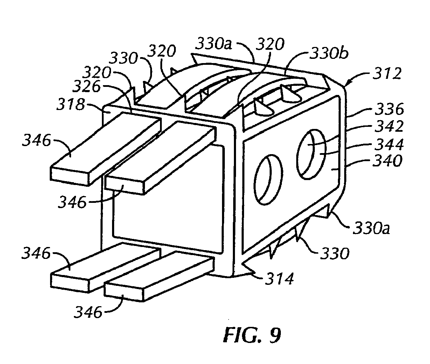

Referring to Fig. 9, the vertebral fixation device 310 may include one or more protective covers 346. The covers 346 are inserted through the slots 326 in the flange 318 and cover the gaps 328 during insertion of the vertebral fixation device 310. The thickness of the covers 346 is sufficient to limit the exposure of the projections 330. When the vertebral fixation device 310 is impacted, the covers 346 allow for smooth insertion by distracting the adjacent vertebrae 102. The covers 346 also prevent debris from entering the central cavity 342 or interior 332 during insertion of the vertebral fixation device 310 and protect nearby anatomic structures, especially the adjacent vertebral bone surfaces, from injury by the projections 330. The protective covers 346 may curve outwardly as shown in Fig. 9 and in phantom in Fig. 7B. Additionally, the protective covers 346 may be part of or otherwise attached to the insertion tool such that the protective covers 346 are removed by or with the insertion tool.

During the insertion of the vertebral fixation device 310 into the disk space using an insertion tool (not shown), two pairs of protective covers 346 are preferably used to cover the gaps 328 and to limit the exposure of the projections 330. Each of the protective covers 346 is identical and is generally in the form of a smooth surfaced stick. The protective covers 346 are of a thickness that matches the height of the projections 330, 320 and sharpened edge 330a so that preferably no sharp point or surface of the projections 330, 320 or the sharpened edges 330a protrudes beyond the smooth surface of the protective covers 346. The sharpened edges 320 may extend beyond the protective covers 346 so that they may be partially driven into the anterior margins of the adjacent vertebral bodies 108. The covers 346 extend through the slots 326 in the proximal end 314.

The insertion tool may be formed of any substantially rigid material, but preferably is formed of titanium, hardened stainless steel, or a biocompatible alloy, composite, polymeric material or the like of sufficient strength. It should be noted that the material of construction of the insertion tool could be any material without diverging from the broad scope of the present invention. The protective covers 346 are preferably made of a biocompatible polymer that is strong and somewhat flexible. However, other materials could be used, such as low density metal alloys, without departing from the spirit or scope of the invention.

During the insertion procedure, an incision is made in an anterior region of a patient proximate a small gap between a first cervical vertebra and a second cervical vertebra 102 of a spine 100 of the patient. Distraction pins (not shown) are inserted into the vertebral bodies 108 above and below the disk space 130' specified for fusion. A distal end of a surgical instrument in inserted to remove disk material 128' from an intervertebral space 130' between the first and second vertebrae 102. The adjacent vertebrae 102 are then prepared for fusion, particularly proximate the anterior margins 108a to accept the vertebral fixation device 310 such that the vertebral fixation device 310 does not protrude beyond the anterior vertebral margins 108a. The intervertebral space 130' may then be measured such that the appropriately sized vertebral fixation device 310 is selected. Once the appropriately sized vertebral fixation device 310 is selected, the vertebral fixation device 310 having the protective covers 346 is inserted into the intervertebral space 130' between the first and second vertebrae 102 such that the proximal end 314 is generally flush with an anterior edge of the first and second vertebrae 102. The protective covers 346 are removed to expose an interior 342 of the vertebral fixation device 310 and the at least one projection 330 extending from the vertebral fixation device 310. The at least one projection 320, 330 and edges 330a engage the bone to hold the vertebral fixation device 310 in place. A solid bone graft or the insert 338 containing bone or non bone material is inserted into the vertebral fixation device 310 to promote fusion. The insert 338 may be inserted before insertion of the frame 312.

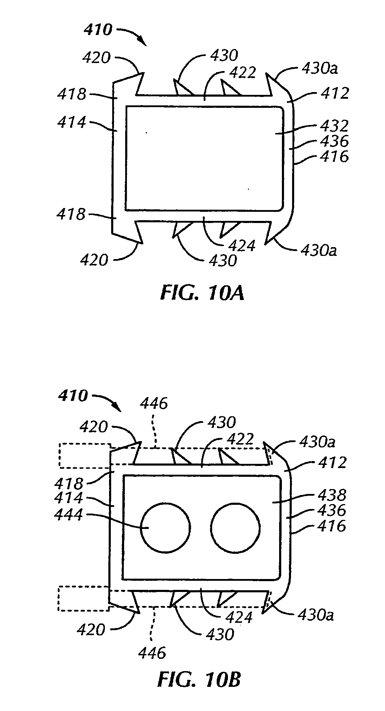

Referring to Figs. 10A-10B, the fourth preferred embodiment of the vertebral fixation device 410, for insertion between a pair of adjacent cervical vertebrae 102, includes generally parallel upper and lower arches 422, 424. The height of the proximal end 414 as measured between the top and the bottom is generally equal to the height of the distal end 416 as measured between the top and the bottom. The vertebral fixation device 410 of the second preferred embodiment is nearly identical to the third embodiment of the vertebral fixation device 310 except that the upper and lower arches 422, 424 form a generally rectangular shape when viewed from the side. Similar numbers indicate similar elements as discussed above for the third embodiment. A discussion of the similar features has been eliminated for convenience only and is not limiting.

It will be appreciated by those skilled in the art that changes could be made to the embodiments described above without departing from the broad inventive concept thereof. It is expected that materials science will create polymers that will allow the combination of fixation, support strength, and subsidence prevention which are embodied in the invention and thus new materials could be used in a single composition without departing from the spirit and scope of the present invention. It is understood, therefore, that this invention is not limited to the particular embodiments disclosed, but it is intended to cover modifications within the spirit and scope of the present invention as defined by the appended claims.
The preferred features of the present invention can be summarized as follows:
1. A dual composition vertebral fixation device for insertion between a pair of adjacent lumbar or thoracic vertebrae, the dual composition vertebral fixation device comprising:
   a frame comprised of a generally rigid first material for structural support strength and rapid fixation having an upper portion and a lower portion, the upper and lower portions being partially convex, spaced from each other and joined together by distal and proximal supports; and
   a housing comprised of a second material for minimizing interference with imaging having a convexly tapered distal end and two generally parallel side walls spaced from each other and extending from the tapered distal end, the housing being coupled to the frame by cooperatively surrounding at least a portion of the frame and oriented such that the side walls are generally perpendicular to the upper and lower portions of the frame when assembled.
2. The dual composition vertebral fixation device of Aspect 1, wherein the first material is formed at least partially of a biocompatible material selected from the group consisting of: machined bone, titanium, a nickel plated metal, a biocompatible alloy, a biocompatible ceramic, a biocompatible polymeric material and a biologically absorbable material.
3. The dual composition vertebral fixation device of Aspect 1, wherein the upper portion of the frame has an upper opening to encourage vertebral fusion and fixation within an intervertebral space.
4. The dual composition vertebral fixation device of Aspect 3, wherein the frame includes a removable protective cover that covers the upper opening during insertion of the dual composition vertebral fixation device within the intervertebral space.
5. The dual composition vertebral fixation device of Aspect 3, wherein the upper potion of the frame includes at least one arch spanning the upper opening.
6. The dual composition vertebral fixation device of Aspect 3, wherein the upper opening has an at least partially sharpened edge.
7. The dual composition vertebral fixation device of Aspect 5, wherein the upper portion of the frame includes at least one sharpened projection extending from the at least one arch.
8. The dual composition vertebral fixation device of Aspect 1, wherein the lower portion of the frame has a lower opening to encourage vertebral fusion and fixation within an intervertebral space.
9. The dual composition vertebral fixation device of Aspect 8, wherein the frame includes a removable protective cover that covers the lower opening during insertion of the dual composition vertebral fixation device within the intervertebral space.
10. The dual composition vertebral fixation device of Aspect 8, wherein the lower portion of the frame includes at least one arch spanning the lower opening.
11. The dual composition vertebral fixation device of Aspect 8, wherein the lower opening has an at least partially sharpened edge.
12. The dual composition vertebral fixation device of Aspect 10, wherein the lower portion of the frame includes at least one sharpened projection extending from the at least one arch.
13. The dual composition vertebral fixation device of Aspect 1, wherein the upper and lower portions are at least partially convexly shaped such that the height of the vertebral fixation device is greatest in an area located between the proximal and distal ends.
14. The dual composition vertebral fixation device of Aspect 1, wherein the proximal support includes a connector port configured to temporarily and removably couple with an insertion tool.
15. The dual composition vertebral fixation device of Aspect 14, wherein the connector port is one of a threaded opening, a socket, a detent, a hole and a slot
16. The dual composition vertebral fixation device of Aspect 1, wherein the second material is formed of at least partially of a biocompatible material selected from the group consisting of: a ceramic, a polymeric material and a biologically absorbable material.
17. The dual composition vertebral fixation device of Aspect 16, wherein the second material is PEEK.
18. The dual composition vertebral fixation device of Aspect 1, wherein each of the sides of the housing includes at least one opening.
19. The dual composition vertebral fixation device of Aspect 1, wherein the housing includes at least one tab that is received into the proximal support when the housing and frame are assembled.
20. The dual composition vertebral fixation device of Aspect 1, wherein at least one fastener connects the housing to the frame.
21. The dual composition vertebral fixation device of Aspect 1, further comprising an interior space generally defined by the upper and lower portions, the proximal and distal supports, and the side walls of the housing.
22. The dual composition vertebral fixation device of Aspect 21, wherein the interior space houses bone grafts.
23. The dual composition vertebral fixation device of Aspect 21, wherein the interior space houses non-bone matter.
24. The dual composition vertebral fixation device of Aspect 1, wherein the assembled vertebral fixation device is at least partially convex such that the side walls located proximate the center of the vertebral fixation device are the widest lateral part of the vertebral fixation device, the side walls taper toward the proximal and distal ends.
25. The dual composition vertebral fixation device of Aspect 24, wherein the height of the vertebral fixation device is different than the width.
26. The dual composition vertebral fixation device of Aspect 1, wherein both the proximal and the distal ends of the vertebral fixation device are generally convexly tapered and the proximal end has a lesser average radius of curvature than the distal end.
27. The dual composition vertebral fixation device of Aspect 1, wherein the side walls are at least partially planar and generally perpendicular to the upper and lower portions.
28. The dual composition vertebral fixation device according to Aspect 1, wherein the length of the housing as measured from the distal end to proximal support of the frame is greater than the width of the housing as measured between the sides of the housing and is greater than the height of the housing as measured between the upper and lower portions of the frame.
29. The dual composition vertebral fixation device according to Aspect 1, wherein the distal end of the vertebral fixation device has a taper such that maximum transverse dimension changes by less than 2 mm for every 1 mm change in length.
30. The dual composition vertebral fixation device according to Aspect 1, wherein a terminal distal 1 mm of the vertebral fixation device measuring along a longitudinal axis has a maximum transverse dimension of 15-20% of the maximum height of the vertebral fixation device.
31. A method of installing a vertebral fixation device, the method comprising:
   a) making an incision in a posterior region of a patient proximate a small gap between a first vertebra and a second vertebra of a spine of the patient,
   c) inserting a distal end of a surgical instrument through the small gap between the first and second vertebrae in order to access an intervertebral space between the first and second vertebrae;
   d) removing nuclear disk material from the intervertebral space;
   e) inserting the vertebral fixation device with protective covers, the vertebral fixation device having a frame comprised of a rigid first material for structural support strength and rapid fixation and a housing comprised of a second material for minimizing interference with imaging having a convexly tapered distal end, through the small gap, the tapered distal end separating the first and second vertebrae as the vertebral device is inserted into and within a nuclear region of the intervertebral space between the first and second vertebrae; and
   f) removing the protective covers to expose an interior of the vertebral fixation device and at least one projection and at least one sharpened edge extending from the vertebral fixation device.
32. The method ofAspect 31, wherein the incision is off-center with respect to the posterior-side of the spine of the patient and proximate to a neuroforaminae between the first and second vertebrae.
33. The method of Aspect 31, wherein a distal end of a working channel is inserted through the incision.
34. A vertebral fixation device for insertion between a pair of adjacent cervical vertebrae, the vertebral fixation device comprising:
   a frame comprised of a first material and having a generally rectangular proximal end and a generally rectangular distal end, the distal end connected to the proximal end by at least one upper arch and at least one lower arch, the upper and lower arches being spaced apart from each other, the frame having a generally hollow interior and having substantially open lateral sides between the upper and lower arches and a substantially open proximal end.
35. The vertebral fixation device of Aspect34, wherein the frame tapers from the proximal end toward the distal end between the upper and lower arches such that the distance between the upper and lower arches decreases from the proximal end toward the distal end.
36. The vertebral fixation device of Aspect 34, wherein the first material is formed at least partially of a generally rigid biocompatible material selected from the group consisting of: machined bone, titanium, a nickel plated metal, an alloy, a ceramic, a polymeric material and a biologically absorbable material.
37. The vertebral fixation device of Aspect 34, further including an insert comprised of a second material, the insert forming a box-shaped structure similar to the hollow interior of the frame, the insert disposed within at least a portion of the frame and having lateral sides exposed at least partially through the open lateral sides of the frame, the insert having a generally hollow interior.
38. The vertebral fixation device of Aspect 37, wherein the second material is formed of at least partially of a biocompatible material selected from the group consisting of: a ceramic, a polymeric material and a biologically absorbable material.
39. The vertebral fixation device of Aspect 37, wherein the exposed lateral sides of the insert each includes at least one opening.
40. The vertebral fixation device of Aspect 34, wherein the proximal end of the frame includes at least one flange.
41. The vertebral fixation device of Aspect 40, wherein the at least one flange includes at least one sharpened edge.
42. The vertebral fixation device of Aspect 41, wherein the at least one sharpened edge projects toward the distal end of the vertebral fixation device.
43. The vertebral fixation device of Aspect 34, further comprising an interior that is generally defined by a shape of the frame.
44. The vertebral fixation device of Aspect 43, wherein the interior houses at least one bone graft.
45. The vertebral fixation device of Aspect43, wherein the interior houses non-bone matter.
46. The vertebral fixation device of Aspect 34, wherein the upper arch of the frame is proximate to a gap configured to promote bone growth within an intervertebral space.
47. The vertebral fixation device of Aspect 46, wherein the frame includes at least one upper protective cover that covers the gap during insertion of the vertebral fixation device.
48. The vertebral fixation device of Aspect 34, wherein the at least one upper arch has an at least one partially sharpened projection extending therefrom.
49. The vertebral fixation device of Aspect 48, wherein the at least one partially sharpened projection extends toward the proximal end of the vertebral fixation device.
50. The vertebral fixation device of Aspect 34, wherein the distal end of the at least one upper arch has a sharpened edge, the sharpened edge extends toward the proximal end of the vertebral fixation device.
51. The vertebral fixation device of Aspect 34, wherein the lower arch of the frame is proximate to a gap configured to promote bone growth within an intervertebral space.
52. The vertebral fixation device of Aspect 51, wherein the frame includes at least one lower protective cover that covers the gap during insertion of the vertebral fixation device.
53. The vertebral fixation device of Aspect34, wherein the at least one lower arch has an at least one partially sharpened projection extending therefrom.
54. The vertebral fixation device of Aspect 53, wherein the at least one partially sharpened projection extends toward the proximal end of the vertebral fixation device.
55. The vertebral fixation device of Aspect 34, wherein the distal end of the at least one lower arch has a sharpened edge, the sharpened edge extends toward the proximal end of the vertebral fixation device.
56. A method of installing a vertebral fixation device, the method comprising:
   a) making an incision in an anterior region of a patient proximate a small gap between a first cervical vertebra and a second cervical vertebra of a spine of the patient;
   b) inserting a distal end of a surgical instrument and removing disk material from an intervertebral space between the first and second vertebrae;
   c) preparing the first and second vertebrae for fusion;
   d) inserting the vertebral fixation device with protective covers into the intervertebral disk space such that the proximal end of the vertebral fixation device is generally flush with an anterior edge of the first and second vertebrae; and
   e) removing the protective covers to expose an interior of the vertebral fixation device and the at least one projection extending from the vertebral fixation device.
57. The method of Aspect56, wherein the at least one projection extending from the vertebral fixation device proximate the proximal end is inserted into the anterior edge of at least one of the first and second vertebrae.

## Claims

1. A vertebral fixation device for insertion between a pair of adjacent cervical vertebrae, the vertebral fixation device comprising:
a frame comprised of a first material and having a generally rectangular proximal end and a generally rectangular distal end, the distal end connected to the proximal end by at least one upper arch and at least one lower arch, the upper and lower arches being spaced apart from each other, the frame having a generally hollow interior and having substantially open lateral sides and a substantially open proximal end and at least one of the upper and lower arches being spaced between the lateral sides of the frame.

2. The vertebral fixation device of claim 1, wherein the frame tapers from the proximal end toward the distal end between the upper and lower arches such that the distance between the upper and lower arches decreases from the proximal end toward the distal end.

3. The vertebral fixation device of claim 1, wherein the first material is formed at least partially of a generally rigid biocompatible material selected from the group consisting of: machined bone, titanium, a nickel plated metal, an alloy, a ceramic, a polymeric material and a biologically absorbable material.

4. The vertebral fixation device of claim 1, further including an insert comprised of a second material, the insert forming a box-shaped structure similar to the hollow interior of the frame, the insert disposed within at least a portion of the frame and having lateral sides exposed at least partially through the open lateral sides of the frame, the insert having a generally hollow interior.

5. The vertebral fixation device of claim 4, wherein the second material is formed at least partially of a biocompatible material selected from the group consisting of: a ceramic, a polymeric material and a biologically absorbable material.

6. The vertebral fixation device of claim 4, wherein the exposed lateral sides of the insert each includes at least one opening.

7. The vertebral fixation device of claim 1, wherein the proximal end of the frame includes at least one flange.

8. The vertebral fixation device of claim 7, wherein the at least one flange includes at least one sharpened edge.

9. The vertebral fixation device of claim 8, wherein the at least one sharpened edge projects toward the distal end of the vertebral fixation device.

10. The vertebral fixation device of claim 1, wherein the interior houses at least one bone graft.

11. The vertebral fixation device of claim 1, wherein at least one of the upper arch and the lower arch of the frame is proximate to a gap configured to promote bone growth within an intervertebral space.

12. The vertebral fixation device of claim 11, wherein the frame includes at least one protective cover that covers the gap during insertion of the vertebral fixation device.

13. The vertebral fixation device of claim 1, wherein at least one of the at least one upper arch and the at least one lower arch has an at least one partially sharpened projection extending therefrom.

14. The vertebral fixation device of claim 13, wherein the at least one partially sharpened projection extends toward the proximal end of the vertebral fixation device.

15. The vertebral fixation device of claim 1, wherein the distal end has a sharpened edge, the sharpened edge extends at least partially between the lateral sides and toward the proximal end of the vertebral fixation device.

16. The vertebral fixation device of claim 1, wherein the distal end has a sharpened edge, the sharpened edge extends at least partially between the lateral sides and toward the proximal end of the vertebral fixation device.
